# EUROPÄISCHE PATENTSCHRIFT

(11) **EP 1 100 396 B1**
(45) Veröffentlichungstag und Bekanntmachung des Hinweises auf die Patenterteilung: **17.12.2003**
(21) Anmeldenummer: 99932596.2
(22) Anmeldetag: 29.07.1999
(51) Int. Cl.: A61C 8/00, A61F 2/42

(54) **IMPLANTAT ZUM HALTEN UND/ODER BILDEN EINES ZAHNERSATZES ODER EINES KÜNSTLICHEN FINGERGELENKS**
IMPLANT FOR HOLDING AND/OR FORMING A DENTAL PROSTHESIS OR ARTIFICIAL FINGER JOINT
IMPLANT POUR MAINTENIR ET/OU FORMER UNE PROTHESE DENTAIRE OU UNE ARTICULATION ARTIFICIELLE DU DOIGT

(30) Priorität: 30.07.1998 CH 159698
(43) Veröffentlichungstag der Anmeldung: 23.05.2001
(73) Patentinhaber: Sutter, Franz, 4435 Niederdorf (CH)
(72) Erfinder: Sutter, Franz, 4435 Niederdorf (CH)
(74) Vertreter: Eder, Carl E.
(86) Internationale Anmeldenummer: CH9900357
(87) Internationale Veröffentlichungsnummer: WO00006043

(56) Entgegenhaltungen:
- EP-A- 0 169 976
- DE-A- 4 342 468
- FR-A- 2 084 522
- GB-A- 2 210 795

## Beschreibung

Die Erfindung betrifft ein Implantat zum Halten und/oder Bilden eines Zahnersatzes oder eines künstlichen Fingergelenks.

Aus den Figuren 14, 15 der US 4 447 209 A ist ein Dentalimplantat mit einer zum Verankern in einem Kieferknochen bestimmten Verankerungspartie bekannt. Diese weist eine im allgemeinen zylindrische Hülse auf, deren Mantelfläche mit axialen, entlang dem Umfang abwechselnd aufeinander folgenden Rippen und Rillen versehen ist. Diese verbessern die Übertragung von quer zur Achsen der Hülse gerichteten Kräften zwischen dem Implantat und dem Knochen, tragen aber nicht nennenswert zur Übertragung von ungefähr achsparallelen Kräften bei. Der Mantel der Hülse ist ferner mit Löchern versehen. Die vielen Löcher, die über die ganze axiale Ausdehnung des Rippen aufweisenden Implantatabschnitts verteilt sind, schwächen jedoch das Implantat und vergrössern dessen Biegbarkeit. Dies ist insbesondere dann der Fall, wenn die zylindrische Hülse nur einen kleinen Durchmesser hat. Bei einem derartigen Implantat besteht eine erhebliche Gefahr, dass das Implantat bei Belastungen Mikrobewegungen ausführt, die in der Umgebung des Implantats einen Knochenabbau und dadurch eine Lockerung des Implantats verursachen. Zudem kann das Implantat bei starken Belastungen bei den sich in der Nähe des Knochenkamms befindenden Löchern brechen.

Ein aus der FR 2 084 522 A bekanntes Implantat besitzt eine im wesentlichen konische Verankerungspartie, die nach der Extraktion eines natürlichen Zahns mit dem dünneren Konusende voran in die frei gewordene Alveole des Kieferknochens eingesetzt wird. Die konische Verankerungspartie ist mit Vorsprüngen versehen. Diese sind vorzugsweise in der vom dünneren Konusende wegverlaufenden Richtung von der konischen Fläche weg nach aussen geneigt und haben an dem dem dünneren Ende abgewandten Ende eine Endfläche. Dagegen laufen sie an ihren sich näher beim dünneren Konusende befindenden Ende mindestens annähernd in eine Spitze aus. Die konischen Verankerungspartien werden vor dem Einsetzen in Knochen zur Anpassung an die individuellen Formen der Alveolen beschliffen. Dies verursacht zusätzliche Arbeit und kann zudem zur Folge haben, dass einige der Vorsprünge weggeschliffen werden. Wegen der im allgemeinen konischen Form der Verankerungspartie sind die Querschnittsformen und Querschnittsabmessungen der Vorsprünge in zur Achse der Verankerungspartie rechtwinkligen Schnitten entlang dieser Achse nicht konstant. Dementsprechend sind die Scheitel, Längsflächen und Längsränder der Vorsprünge nicht oder zumindest nicht alle parallel zur Achse der Verankerungspartie. Ferner haben die sich nahe beim dünneren Konusende befindenden Vorsprünge kleinere radiale Abstände von der Achse als die weiter vom dünneren Konusende entfernten Vorsprünge. Aus diesen Gründen können die Vorsprünge beim Einführen einer Verankerungspartie in einen Knochen nur schwer und höchstens sehr wenig in das Knochenmaterial eindringen und bewirken daher nicht sofort nach dem Einsetzen eine stabile Verankerung. Des weiteren vergrössern die Vorsprünge die Torsionsfestigkeit der Verbindung des Implantats mit dem Knochen auch nach dem Verwachsen des Knochens mit der Verankerungsparie nur wenig. Da die Vorsprünge an ihren sich näher beim dünneren Konusende befindenden Enden keine oder mindestens keine nennenswerten Endflächen haben, tragen sie auch höchstens wenig zur Übertragung von achsparallelen, zum dünneren Konusende hin gerichteten Druckkräften von der Verankerungspartie auf den Knochen bei.

Es sind ferner künstliche Fingergelenke mit zwei schwenkbar miteinander verbundenen Implantaten bzw. Gelenkteilen bekannt. Jedes dieser Implantate hat eine Verankerungspartie und einen Gelenkkopf. Die Verankerungspartie wird bei der Verwendung des Implantats in einem Knochen eines Fingers eingesetzt. Die Verankerungspartie besteht aus einem zylindrischen Bolzen mit einer glatten Oberfläche und ergibt daher nur eine schwache Verankerung im Knochen.

Der Erfindung liegt daher die Aufgabe zugrunde, Nachteile der bekannten Implantate zu vermeiden und ein Implantat zu schaffen, welches schon unmittelbar nach dem Einsetzen in einen Knochen eine stabile Verbindung mit diesem ermöglicht, spätestens nach dem Einheilen sowohl ungefähr quer zur Achse gerichtete Kräfte als auch ungefähr axiale Kräfte gut auf den Knochen übertragen kann und trotzdem auch bei kleinem Durchmesser ausreichend bruchfest ist und eine genügende, günstige Biegesteifigkeit hat.

Diese Aufgabe wird gemäss der Erfindung durch ein Implantat mit den Merkmalen des Anspruchs 1 gelöst. Vorteilhafte Ausgestaltungen des Implantats gehen aus den abhängigen Ansprüchen hervor.

Die Verankerungspartie des erfindungsgemässen Implantats kann bei der Verwendung des letzteren parallel zur Achse der Verankerungspartie derart in ein im wesentlichen zylindrisches Loch eines Knochens eingesetzt werden, dass die Vorsprünge im Querschnitt teilweise in das Knochenmaterial hineingedrückt werden und dass dementsprechend Knochenmaterial in die zwischen benachbarten Vorsprüngen vorhandenen, axialen Zwischenräume bzw. Rillen hineinragt und diese vorzugsweise teilweise oder eventuell sogar vollständig ausfüllt. Das an die Verankerungspartie angrenzende Knochenmaterial wird beim Hineindrücken des Implantats ein wenig zerspant und/oder verdichtet. Die Verdichtung ist insbesondere dann von Vorteil, wenn das an die Verankerungspartie angrenzende Knochenmaterial teilweise aus relativ poröser Spongiosa besteht. Die Verankerungspartie wird durch das Einpressen beim Einsetzen des Implantats sofort ziemlich stabil verankert und erhält dadurch sofort eine gute Stabilität, die sogenannte Primärstabilität. Beim Einheilen wächst das Knochenmaterial in die oder jede Ausnehmung bzw. Nut hinein, die zwischen axial voneinander in Abstand stehenden Vorsprüngen vorhanden ist. Ferner werden die vorzugsweise unmittelbar nach dem Einsetzen des Implantats nur teilweise mit Knochenmaterial gefüllten, axialen Zwischenräume bzw. Rillen beim Einheilen vollständig mit Knochenmaterial ausgefüllt. Das Implantat wird dann sehr stabil im Knochen verankert und kann sowohl grosse, ungefähr achsparallele Kräfte als auch grosse, ungefähr quer zur Achse gerichtete Kräfte auf den Knochen übertragen. Die Kräfte werden dabei ziemlich gleichmässig auf eine grosse, an das Implantat angrenzende Fläche des Knochens verteilt. Dadurch können auch bei starken Belastungen des Implantats lokale Überbeanspruchungen des Knochens vermieden werden, welche eine Resorption von Knochenmaterial verursachen könnten. Das erfindungsgemässe Implantat kann daher auch dauerhaft verankert werden.

Der Erfindungsgegenstand und weitere Vorteile von diesem werden anschliessend anhand in der Zeichnung dargestellter Ausführungsbeispiele näher erläutert. In der Zeichnung zeigt
Fig. 1 einen Schnitt durch einen Knochen und ein in diesen eingesetztes, einstückiges, in Ansicht gezeichnetes Dentalimplantat mit Rippen, die durch eine schraubenlinienförmige Nut unterteilt sind,
Fig. 2 einen Querschnitt durch das Implantat entlang der Linie II-II der Fig. 1
Fig. 3 einen Querschnitt durch einen Bereich des Implantats gemäss den Figuren 1, 2 und durch einen Knochen unmittelbar nach dem Einsetzen des Implantats,
Fig. 4 eine der Fig. 3 entsprechende Darstellung, aber nachdem der Knochen mit dem Implantat verwachsen ist,
Fig. 5 eine schematische Darstellung eines Teils des Implantats und der Übertragung einer ungefähr axialen Kraft,
Fig. 6 einen Querschnitt durch das Implantat gemäss den Figuren 1 bis 5 und eine schematische Darstellung der Übertragung einer quer zur Achse gerichteten Kraft,
Fig. 7 eine zur Fig. 6 analoge Darstellung der Kraftübertragung für ein nicht-erfindungsgemässes Implantat ohne Rippen,
Fig. 8 eine Schrägansicht eines im allgemeinen vollständig zylindrischen, transgingival in einen Knochen eingesetzten Implantats, dessen Rippen durch Ringnuten voneinander getrennt sind,
Fig. 9 einen Querschnitt durch das Implantat gemäss Fig. 8 in grösserem Massstab unmittelbar nach dem Einsetzen des Implantats in den Knochen,
Fig. 10 eine Schrägansicht eines anderen, im allgemeinen vollständig zylindrischen, subgingival in einen Knochen eingesetztes Implantats,
die Figuren 11 bis 13 zur Fig. 5 analoge Darstellungen von Implantaten mit durch Ringnuten unterteilten Rippen mit unterschiedlichen Verhältnissen zwischen den axialen Abmessungen der Rippen und Ringnuten,
Fig. 14 einen in Abwicklung dargestellten Bereich der Umfangsfläche eines Implantats, dessen einander entlang dem Umfang benachbarten Rippen axial gegeneinander versetzt sind,
Fig. 15 eine Schrägansicht eines abgewinkelten Implantats,
Fig. 16 eine Schrägansicht eines Implantats mit einem metallischen Haupt-Körper und einem Keramik-Ring und
Fig. 17 eine Schrägansicht eines künstlichen Fingergelenks mit zwei in Knochen eingesetzten Implantaten.

Zu den nachfolgend beschriebenen Ausführungsbeispielen wird darauf hingewiesen, dass einander entsprechende, identische oder ähnliche Teile der verschiedenen Ausführungsbeispiele jeweils mit der gleichen Bezugsnummer bezeichnet werden.

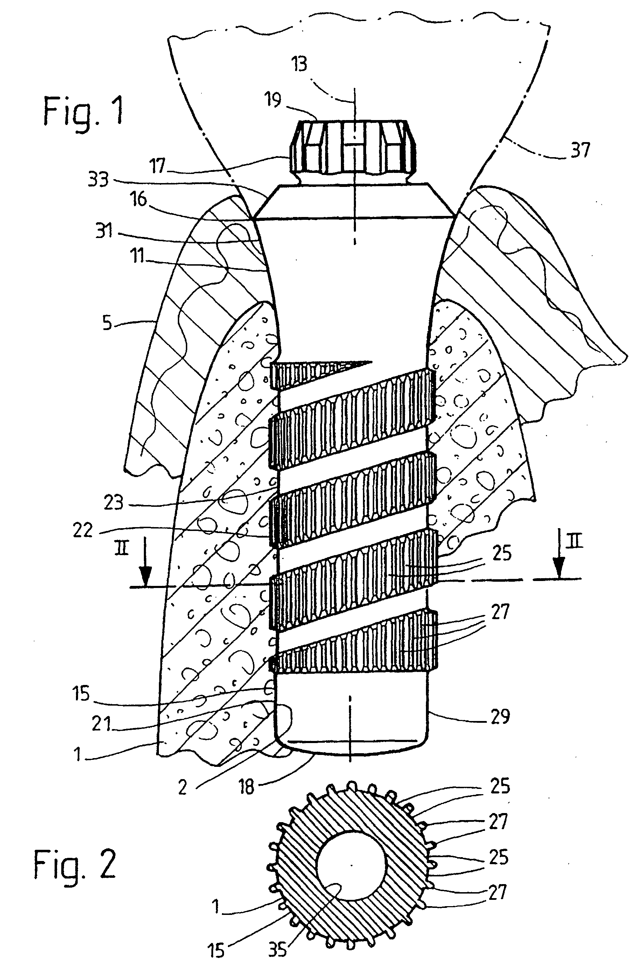

Der in Fig. 1 ersichtliche Knochen 1 gehört beispielsweise zum Unterkiefer eines Patienten und wurde durch Bohren und/oder Fräsen mit einem im wesentlichen zylindrischen Sackloch 2 versehen, das in den Kamm des Knochens 1 mündet. In Fig. 1 ist ferner das den Knochen bedeckende Weichgewebe 5 die Gingiva, ersichtlich.

Ein in den Figuren 1 bis 6 ersichtliches, einstückiges enossales Dentalimplantat 11 zum Halten und/oder Bilden eines Zahnersatzes ist länglich sowie im allgemeinen rotationssymmetrisch zu einer Achse 13. Das Implantat besitzt von unten nach oben eine Verankerungspartie 15, eine Schulter 16 und einen Kopf 17. Das Implantat hat zwei einander abgewandte, freie Enden, nämlich ein von der Verankerungspartie 15 gebildetes, unteres, erstes Implantatende 18 und ein vom Kopf gebildetes, oberes, zweites Implantatende 19. Die Verankerungspartie 15 ist im grössten Teil ihrer axialen Ausdehnung im allgemeinen zylindrisch und hat eine Umfangsfläche 21. Der im allgemeinen zylindrische Abschnitt der Verankerungspartie besitzt jedoch einen schraubenlinienförmigen, verdickten Bereich 22 bzw. eine Verdickung 22. Die Windungen des verdickten Bereichs 22 sind durch eine schraubenlinienförmige Ausnehmung 23 bzw. Nut 23 voneinander getrennt. Der verdickte Bereich 22 und die Nut 23 haben oder bilden je mindestens eine volle, die Achse 13 umschliessende Windung, vorzugsweise mindestens zwei solche Windungen und beispielsweise mindestens drei solche Windungen. Die schraubenlienförmige Nut 23 hat einen Grund, der durch einen Abschnitt der Umfangsfläche 21 gebildet ist. Dieser Umfangsflächenabschnitt ist zur Achse 13 koaxial und mindestens zum Teil sowie zum Beispiel vollständig zylindrisch. Der schraubenlinienförmige Bereich 22 hat einen zur Achse 13 parallelen Scheitel und zwei einander abgewandte Randflächen bzw. Flanken. Der schraubenlinienförmige Bereich ist am unteren Ende sowie am oberen Ende durch einen Rand begrenzt der entlang einer zur Achse 13 rechtwinkligen Ebene verläuft, so dass der schraubenlinienförmige Bereich 22 an beiden Enden in eine Spitze ausläuft.

Der schraubenlinienförmige, verdickte Bereich 22 ist durch gleichmässig um die Achse 13 herum verteilte, zur letzteren parallele Ausnehmungen oder Zwischenräume 25 in Vorsprünge 27 unterteilt. Der schraubenlinienförmigen Bereich 22 hat also um die Achse 13 herum abwechselnd aufeinander folgende Vorsprünge 27 und Ausnehmungen oder Zwischenräume 25. Die Ausnehmungen oder Zwischenräume 25 und Vorsprünge 27 sind - ausgenommen bei den auslaufenden Enden des Bereichs 22 - länglich und durch axiale, d.h. zur Achse 13 parallele, Rillen 25 bzw. Rippen 27 gebildet. Die durch Windungen der schraublinienförmigen Ausnehmung bzw. Nut 23 voneinander getrennten Vorsprünge bzw. Rippen 27 fluchten gruppenweise miteinander und bilden gerade, zur Achse 13 parallele Reihen. Eine volle Windung des Bereichs 22 weist mindestens 6, vorzugsweise mindestens 10, noch besser mindestens 15 oder mindestens 18, vorzugsweise höchstens 36 und zum Beispiel 24 um die Achse herum verteilte Ausnehmungen oder Zwischenräume bzw. Rillen 25 und selbstverständlich die gleiche Anzahl Vorsprünge bzw. Rippen 27 auf. Die axialen Rillen 25 sind beispielsweise ungefähr gleich tief wie die schraubenlinienförmige Nut 23 oder eventuell etwas weniger tief als die letztere und haben einen Grund, der einen streifenförmigen Abschnitt einer zur Achse 13 koaxialen Zylinderfläche oder eine schmale, ebene Fläche bildet und mindestens annähernd glatt an den Grund der schraubenlinienförmigen Nut 23 anschliesst.

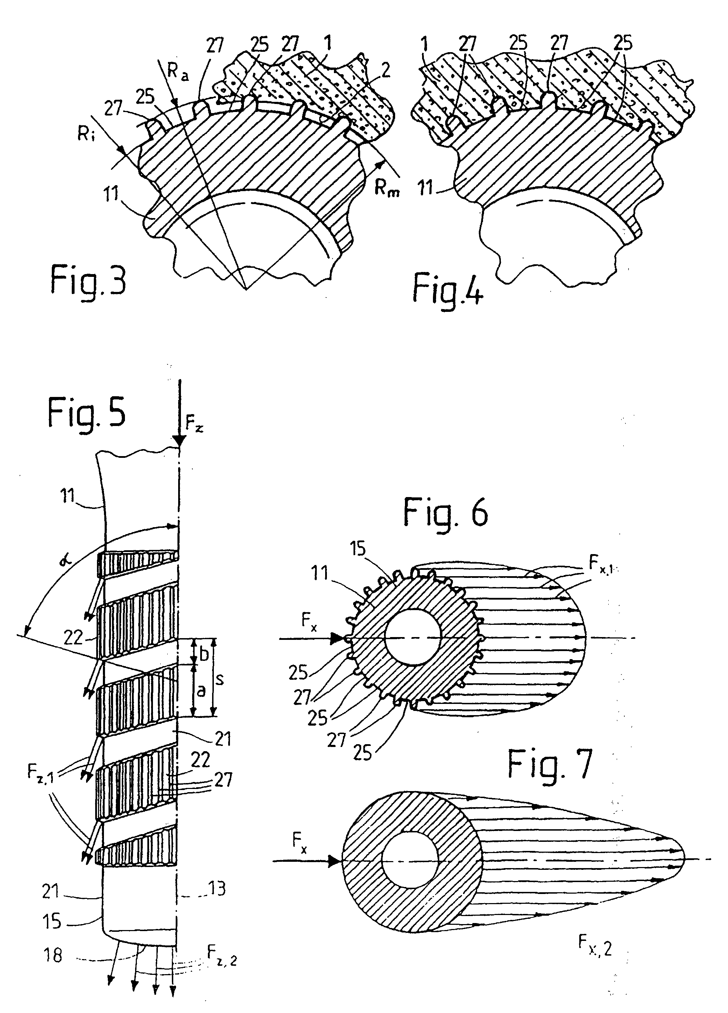

Wie es in Fig. 2 und besonders deutlich in den Figuren 3 und 4 ersichtlich ist, hat jede Rippe 27 zwei ebene zur Achse 13 und zu einander parallele Flanken und einen im Querschnitt konvex gebogenen Scheitel, der die beiden Flanken glatt und stetig miteinander verbindet. Zwischen den Flanken der Rippen und den Grundflächen der Rillen 25 sind beispielsweise gemäss den Figuren 2 bis 4 mehr oder weniger scharfe Kanten vorhanden. Diese könnten jedoch durch gebogene Übergänge ersetzt werden, welche die Flanken der Rippen und Grundflächen der Rillen stetig miteinander verbinden. Die Scheitel, Flanken und Längsränder der Rippen sind gerade und parallel zur Achse 13. Die Scheitel oder - genauer gesagt - die bei den höchsten Stellen der Rippen vorhandenen Scheitellinien definieren zusammen eine für alle Rippen gemeinsame Zylinderfläche. Die Scheitellinien aller Rippen haben dementsprechend den gleichen radialen Abstand von der Achse 13.

Jede Rippe 27 hat zwei einander abgewandte Endflächen, die durch die beiden Randflächen bzw. Flanken des schraubenlinienförmigen Bereichs 22 gebildet sind. Die sich näher beim ersten Implantatende 18 befindenden Endflächen der Rippen sind in zur Achse 13 parallelen Schnitten gerade und bilden in diesen Schnitten mit der Achse 13 einen Winkel, der in Fig. 5 mit α bezeichnet ist. Diese Endflächen sind mindestens im Abschnitt des Bereichs 22, in dem der untere Rand des Bereichs 22 an die Nut 23 angrenzt und schraubenlinienförmig ist, im Axialschnitt von der Achse 13 weg nach aussen vom ersten Implantatende weg geneigt. Der Winkel α beträgt mindestens im genannten Abschnitt des Bereichs 22 vorzugsweise mindestens 60° und zum Beispiel ungefähr 70° bis 80°. Die dem ersten Implantatende 18 abgewandten Endflächen der Rippen 27 sind zum Beispiel mindestens in demjenigen Abschnitt des Bereichs 22, in dem die letztgenannten Endflächen an die schraubenlinienförmige Nut 23 angrenzen, in Axialschnitten ebenfalls gerade und von der Achse weg nach aussen zum ersten Implantatende 18 hin geneigt, wobei sie mit der Achse einen Winkel bilden, der beispielsweise gleich gross ist wie der Winkel α. Jede Endfläche einer Rippe ist von der bzw. jeder Längsfläche der Rippe durch eine zum Beispiel einigermassen scharfe Kante abgegrenzt. Die Rippen 27 haben in zur Achse 13 rechtwinkligen Querschnitten im wesentlichen über ihre ganze Länge - nämlich von einer geneigten Endfläche zur gegenüberstehenden, geneigten Endfläche - konstante Querschnittsabmessungen. Ferner haben alle Rippen die gleichen Querschnittsformen und die gleichen Querschnittsabmessungen.

Die Steigung der schraubenlinienförmigen Bereichs 22 ist in Fig. 5 mit s bezeichnet und beträgt bei einem Dentalimplantat vorzugsweise 1 mm bis 3 mm und zum Beispiel 1,5 mm bis 2,5 mm. Im oben und unten durch einen schraubenlinienförmigen Rand begrenzten Hauptabschnitt des Bereichs 22 haben dieser und dessen Rippen 27 eine Länge oder axiale Abmessung a. Die schraubenlinienförmige Nut 23 hat in Axialschnitten eine in axialer Richtung gemessene Abmessung (oder axiale Breite) b. Da die Rippen 27 geneigte Endflächen haben, werden die Abmessungen a und b in der halben Höhe der Rippen 27 gemessen. Die Länge bzw. axiale Abmessung a der Rippen beträgt mindestens 20%, vorzugsweise mindestens 50% und noch besser mehr als 50% der Steigung s, nämlich zum Beispiel 60% bis 80% der Steigung s. Die Abmessung b der Nut 23 ist selbstverständlich gleich der Differenz s - a. Wenn die Abmessung a des verdickten Bereichs 22 und der Rippen 27 mehr als 50% der Steigung s beträgt, ist die Abmessung a dementsprechend grösser als die im Axialschnitt gemessene Abmessung b der Nut. Die sich an die Scheitel der Rippen 27 anschmiegende, zylindrische Hüllfläche hat einen Radius, der in Fig. 3 mit Rₐ bezeichnet ist. Die Grundflächen der axialen Rillen 25 definieren eine Zylinderfläche mit einem Radius Rᵢ. Die radial gemessene Tiefe der schraubenlinienförmigen Nut 23 sowie der axialen Rillen 25 bzw. die radialen Höhen der Rippen 27 betragen beispielsweise 0,15 mm bis 0,5 mm und beispielsweise mindestens 10% sowie höchstens 35% vom Radius Rₐ. Der sich vom unteren Ende bis zum oberen Ende des schraubenlinienförmigen Bereichs 22 erstreckende Abschnitt der Verankerungspartie hat eine Oberfläche, die infolge der Nut 23 und der Rillen 25 mindestens 30% und beispielsweise ungefähr oder mindestens 50% grösser ist als eine zylindrische, sich an die Scheitel der Rippen anschmiegende, über die axiale Abmessung des verdickten Bereichs 22 erstreckende Hüllfläche.

Die Umfangsfläche der Verankerungspartie 15 hat zwischen dem ersten, unteren Implantatende 18 und dem sich näher bei diesem befindenden, unteren Ende des verdickten Bereichs 22 beipielsweise noch einen glatten zylindrischen Endabschnitt 29 mit dem gleichen Durchmesser wie die Grundfläche der schraubenlinienförmigen Nut. Das Implantat hat oberhalb des verdickten Bereichs 22 einen sich nach oben bis zur Schulter 16 hin trompetenförmig erweiternden Abschnitt 31, der beim oberen Ende der Nut 23 stetig mit deren Grund zusammenhängt. Die Schulter 16 hat eine konische Schulterfläche 33, die sich zum Kopf 17 hin verjüngt. Das Implantat besitzt ferner ein axiales Sackloch 35, das in das vom Kopf 17 gebildete, zweite Implantatende 19 mündet und beispielsweise einen Abschnitt mit einem Innengewinde aufweist. Das Implantat 11 und insbesondere dessen Verankerungspartie 15 sind frei von radialen oder sonst ungefähr quer zur Achse 13 verlaufenden Löchern.

Das Implantat besteht aus einem metallischen Material, beispielsweise Titan. Der sich vom ersten Implantatende 18 bis zum oberen Ende des verdickten Bereichs 22 erstreckende Bereich der Umfangsfläche 21 und die am ersten Implantatende 18 vorhandene Endfläche des Implantats sind eventuell durch eine chemische Behandlung aufgerauht oder mit einem rauhen, aufgesprühten Titanüberzug versehen und haben dann feine, in einem mikroskopischen Grössenbereich liegende Poren.

Zum Einsetzen des Implantats 11 in den Knochen 1 wird dieser mit dem bereits erwähnten Sackloch 2 versehen. Dieses wird derart gebohrt und/oder gefräst, dass es einen Radius Rₘ hat, der kleiner als der Radius Rₐ und mindestens gleich dem Radius Rᵢ sowie vorzugsweise grösser als dieser ist. Ein Zahnarzt kann dann die Verankerungspartie 15 parallel zur Achse des Lochs 2 und der mit dieser zusammenfallenden Achse 13 des Implantats in das Loch 2 hineindrücken. Dabei wird durch die sich näher beim ersten Implantatende 18 befindenden Endflächen der Rippen 27 der untersten Windung der Verdickung 22 eventuell ein wenig Knochenmaterial zerspant, so dass in der vorher zylindrischen Begrenzungsfläche des Sacklochs 2 des Knochens 1 axiale Rillen entstehen. Ferner wird das Knochenmaterial bei den Rippen in radialer Richtung nach aussen gedrückt und etwas verdichtet. Die äusseren Abschnitte der Rippen 27 dringen also beim Einsetzen des Implantats in den Knochen gemäss der Fig. 3 in das Knochenmaterial hinein. Das erste Implantatende 18 des Implantats bildet nach dem Einsetzen des letzteren die sich am tiefsten im Knochen befindende Stelle des Implantats. Ferner befindet sich auch der ganze Bereich 22 innerhalb des Knochens. Das Knochenmaterial füllt nach dem Einsetzen des Implantats die äusseren Bereiche der Rillen 25. Das Implantat wird dadurch beim Einsetzen sofort stabil im Knochen verankert und hat also eine gute Primärstabilität. Ferner fördert die beim Einpressen des Implantats stellenweise stattfindende, leichte Kompression des Knochens das Nachwachsen von Knochenmaterial.

Das Implantat 11 ist gemäss der Fig. 1 transgingival eingesetzt, so dass es aus dem Knochen 1 herausragt und das Weichgewebe 5, d.h. die Gingiva, durchdringt. Das obere, zweite, sich oberhalb des Knochens befindende Implantatende 19 wird dann in üblicher Weise mit einer Einheilkappe verschlossen, damit das Implantat im Knochen einheilen kann. Der Knochen wächst dabei in die schraubenlinienförmige Nut 23 und - gemäss Fig. 4 - in die axialen Rillen 25 hinein, so dass das Knochenmaterial die Nut 23 und die Rillen 25 nach einer beispielsweise etwa 3 Monate dauernden Einheilphase vollständig ausfüllt. Wenn das Implantat im Knochen eingeheilt ist, wird die Einheilkappe entfernt und ein strichpunktiert in Fig. 1 angedeutetes Aufbauelement 37 bzw. eine Suprakonstruktion auf das Implantat 1 aufgebaut und im Sackloch 35 befestigt. Das Aufbauelement bzw. die Suprakonstruktion kann beispielsweise aus einer Krone für einen Einzelzahn oder aus mindestens einem Teil einer Brücke oder mindestens einen Teil einer mehrere künstliche Zähne aufweisenden Zahnprothese bestehen und bildet dann zusammen mit dem Implantat oder eventuell zusammen mit noch anderen Implantaten einen Zahnersatz.

Wenn ein Patient mit dem vom Implantat gehaltenen und/oder gebildeten Zahnersatz kaut, werden unter anderem mehr oder weniger zur Achse 13 parallele Kräfte auf das Implantat 11 ausgeübt. In Fig. 5 ist eine solche axiale Kraft F_{z}, nämlich eine zum ersten Implantatende 18 hin gerichtete Druckkraft durch einen Pfeil bzw. Vektor dargestellt. Diese Kraft F_{z} wird von der Verankerungspartie 15 des Implantats 11 auf den Knochen 1 übertragen und dabei in Teil-Kräfte zerlegt. Dabei werden insbesondere Teil-Kräfte F_{z,1} bei den unteren Endflächen der Vorsprünge bzw. Rippen 27 und Teil-Kräfte F_{z,2} beim ersten Implantatende auf den Knochen übertragen. Die Neigung der unteren Rippen-Endflächen trägt dabei zu einer günstigen Einleitung der Teil-Kräfte F_{z,1} in den Knochen bei.

Beim Kauen werden auch ungefähr quer zur Achse 13 gerichtete Kräfte oder Kraftkomponenten auf das Implantat ausgeübt. Eine solche quer zur Achse 13 gerichtete Kraft Fₓ ist in Fig. 6 durch einen Pfeil bzw. Vektor dargestellt. Die Kraft Fₓ wird von der Verankerungspartie 15 auf den Knochen übertragen und dabei ebenfalls in Teil-Kräfte zerlegt. Dabei wird insbesondere von jeder Rippe 27 eine Teil-Kraft F_{x, 1} auf den Knochen übertragen. Die Kraft Fₓ wird daher bei der Übertragung auf den Knochen relativ gleichmässig über einen zur Kraftrichtung rechtwinkligen Durchmesser bzw. einen Halbkreis verteilt. Da die Endflächen der Rippen entlang einer Schraubenlinie verlaufen und in radialer Blickrichtung gegen eine zur Achse 13 senkrechte Ebene geneigt sind, können die Endflächen der Rippen auch noch quer zur Achse 13 gerichtete Kräfte auf den Knochen übertragen.

Es werden daher sowohl axiale als auch quer zur Achse 13 gerichtete Kräfte oder Kraftkomponenten und dementsprechend Kräfte mit beliebigen Richtungen relativ gleichmässig auf grosse, an das Implantat angrenzende Flächen des Knochens verteilt und auf den letzteren übertragen. Dies gewährleistet auch bei grossen zu übertragenden Kräften, dass weder eine Resorption von Knochenmaterial noch eine Lockerung des Implantats stattfindet. Wenn auf das Implantat bezüglich der Achse 13 ein Drehmoment ausgeübt wird, vergrössern die Rippen 27 und deren entlang einer Schraubenlinie verlaufenden Endflächen den Widerstand gegen eine Drehung des Implantats um die Achse 13 und also die Torsionsfestigkeit der Verankerung.

Jedem Abschnitt der schraubenlinienförmigen Nut 23 steht ein Abschnitt des verdickten, schraubenlinienförmigen Bereichs 22 gegenüber. Wie erwähnt, ist die Abmessung a der Rippen 27 und des Bereichs 22 zudem grösser als die Abmessung b der Nut 23. Die Nut 23 verursacht daher keine nennenswerte Schwächung der Verankerungspartie. Da das Implantat 1 und insbesondere dessen Verankerungspartie ferner keine quer zur Achse 13 verlaufenden Löcher besitzt, hat das Implantat auch bei einem kleinen Durchmesser der Verankerungspartie eine hohe Festigkeit und Biegesteifigkeit.

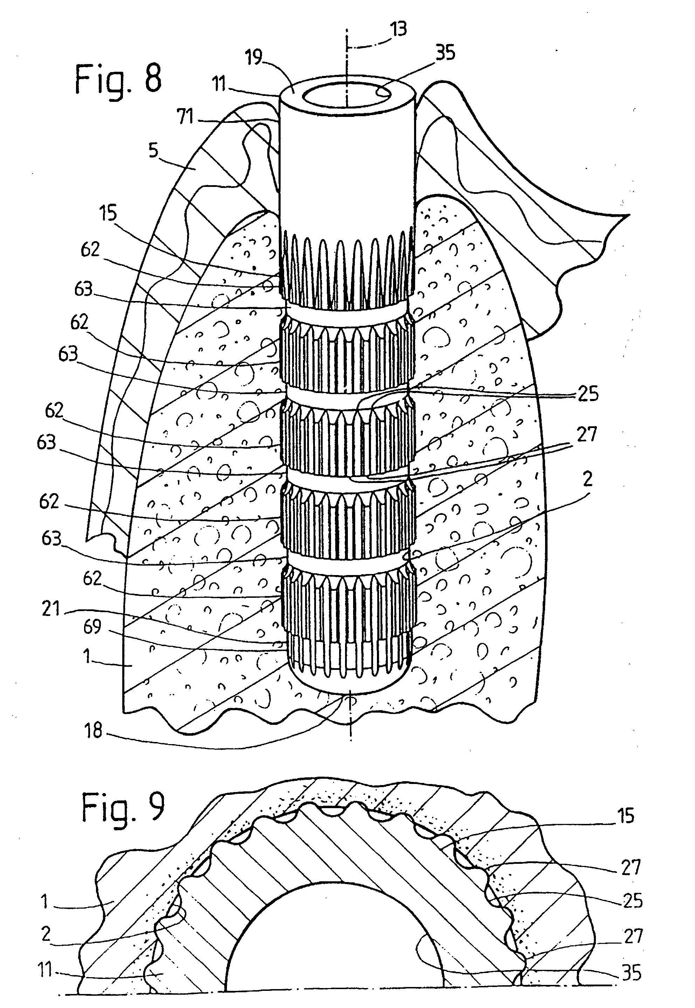

Die Verankerungspartie des in Fig. 7 dargestellten, nicht-erfindungsgemässen Implantats besitzt keine den Rillen 25 und Rippen 27 entsprechenden Rillen und Rippen, sondern lediglich eine glatte Zylinderfläche. Wenn ein zur Achse der Verankerungspartie senkrechte Kraft Fₓ an einem solchen Implantat angreift, wird diese in Teil-Kräfte F_{x, 1} zerlegt, die über einen zur Richtung der Kraft Fₓ senkrechten Durchmesser stark variieren. Ferner wird die Kraft Fₓ auf eine kleinere Oberfläche verteilt als bei einem erfindungsgemässen Implantat mit gleichem Durchmesser der Verankerungspartie. Des weiteren werden bei einem nicht-erfindungsgemässen Implantat, das eine Verankerungspartie mit einer glatten Zylinderfläche aufweist, natürlich auch axiale Kräfte auf eine kleinere Fläche verteilt als bei einem erfindungsgemässen Implantat, dessen Verankerungspartie den gleichen Durchmesser hat.

Nach dem Einheilvorgang schmiegen sich der Knochen 1 und das Weichgewebe 5 oberhalb des Rippen und Rillen aufweisenden, verdickten Bereichs 22 an die zur Achse 13 rotationssymmetrische und glatte Umfangsfläche des trompetenförmigen Abschnitts 31 des erfindungsgemässen Implantats an. Das Weichgewebe kann sich zudem noch an das Aufbauelement 37 anschmiegen. Dies gewährleistet einen guten, dichten Abschluss des im Knochen vorhandenen Lochs. Dadurch wird mindestens weitgehend verhindert, dass Mikroorganismen zwischen das Weichgewebe 5 und das Implantat 11 und in das Loch 2 des Knochens 1 eindringen.

In den Figuren 8 und 9 sind ein Knochen 1, Weichgewebe 5 und ein Dentalimplantat 11 mit einer Achse 13 ersichtlich. Bei dieser Variante ist das ganze Implantat im allgemeinen zylindrisch. Die Umfangsfläche 21 der nach dem Einsetzen des Implantats im Loch 2 des Knochens 1 verankerten Verankerungspartie 15 besitzt mindestens zwei ringförmige, die Achse umschliessende Bereiche 62, die durch eine ringförmige Ausnehmung 63, d.h. eine Ringnut 63, voneinander getrennt sind. Die Bereiche 62 sind durch um die Achse 13 herum verteilte Ausnehmungen oder Zwischenräume 25 in Vorsprünge 27 unterteilt. Vorzugsweise sind mindestens drei und noch besser mindestens vier, nämlich zum Beispiel fünf solche ringförmige Bereiche 62 vorhanden, die durch Ringnuten 63 voneinander getrennt sind und in axialer Richtung voneinander in Abstand stehen. Dementsprechend sind dann mindestens zwei Ringnuten 63 vorhanden. Die Zwischenräume 25 und Vorsprünge 27 sind wiederum länglich und bilden zur Achse 13 parallele Rillen 25 bzw. Rippen 27. Jeder Bereich 62 bildet also einen kreisförmigen Kranz von um die Achse 13 herum abwechselnd aufeinanderfolgenden Rillen 25 und Rippen 27. Die Grundflächen der Rillen 25 und die Scheitel der Rippen 27 sind im Querschnitt gemäss der Fig. 9 derart gebogen, dass sie die Flanken der Rippen glatt und stetig miteinander verbinden.

Die Tiefen der axialen Rillen 25 sind gleich den Tiefen der Ringnuten 63 oder geringfügig kleiner als diese. Die Grundflächen der Ringnuten 63 sind durch glatte Zylinderflächen gebildet.

Die Verankerungspartie hat zwischen dem unteren, ersten Implantatende 18 und dem untersten verdickten Bereich 62 bzw. im unteren Teil des letzteren einen im allgemeinen zylindrischen Endabschnitt 69. Dessen Durchmesser ist kleiner als derjenige der durch die Rippen-Scheitel definierten Hüllfläche, aber grösser als der Durchmesser der Zylinderfläche, die durch die tiefsten Stellen der Rillen 25 definiert wird. Der Endabschnitt 69 wird durch die Rillen der untersten Verdickung bzw. Verlängerungen dieser Rillen ebenfalls in rippenförmige Vorsprünge unterteilt, die jedoch deutlich niedriger sind als die sich weiter oben befindenden Rippen. Die Rillen und Rippen des Endabschnitts 69 erstrecken sich bis zu der im Axialschnitt leicht konvex gebogenen Endfläche am ersten Implantatende 18. Das Implantat hat oberhalb des obersten, ringförmigen, axiale Rippen und Rillen aufweisenden Bereichs 62 einen Endabschnitt 71 mit einer sich bis zum zweiten Implantatende 19 erstreckenden, glatten zylindrischen Umfangsfläche. Deren Durchmesser ist gleich dem Durchmesser der sich an die Scheitel der axialen Rippen 27 anschmiegenden, zylindrischen Hüllfläche. Die Umfangsfläche des Endabschnitts 71 schliesst also glatt an die höchsten Stellen der Rippen an. Die ringförmigen Bereiche 62 können als Verdickungen gegenüber den Ringnuten 63 angesehen werden.

Bei dem gemäss den Figuren 8 und 9 ausgebildeten Implantat haben also mindestens noch all diejenigen Vorsprünge bzw. Rippen 27 über ihre ganze Länge konstante und bei allen Rippen identische Querschnittsformen und Querschnittsabmessungen, die sich entlang der Achse zwischen anderen Rippen der gleichen Rippen-Reihe befinden und also weder zum untersten noch zum obersten Rippen-Kranz gehören.

Die Rippen 27 haben mit Ausnahme der zum obersten Bereich 62 gehörenden Rippen an beiden Enden mit der Achse 13 einen Winkel bildende Endflächen. Die Rippen des obersten Bereichs 62 haben nur an ihrem unteren Ende Endflächen. Dafür bilden die Rillen des obersten Bereichs 62 bei ihren oberen Enden nach aussen auslaufende, mit der Achse einen Winkel bildende Endflächen. Im übrigen besitzt das in den Figuren 8 und 9 dargestellte Implantat gleich wie das in den Figuren 1 bis 6 dargestellte Implantat keine quer zur Achse verlaufenden Löcher.

Das Implantat ist gemäss der Fig. 8 derart in das Loch 2 des Knochens 1 eingesetzt, dass der oberste, Rillen und Rippen aufweisende Bereich 62 sich ein wenig unterhalb des Kamms des Knochens 1 befindet. Die Länge des eine glatte, zylindrische Umfangsfläche aufweisenden Endabschnitts 71 ist derart bemessen, dass das obere, zweite Implantatende 19 sich ungefähr in der Höhe des Kamms des Weichgewebes 5 befindet. Im übrigen hat das Implantat wiederum ein axiales Sackloch 35, das in das obere, zweite Implantatende 19 mündet und dort von einer ebenen Ringfläche umschlossen wird.

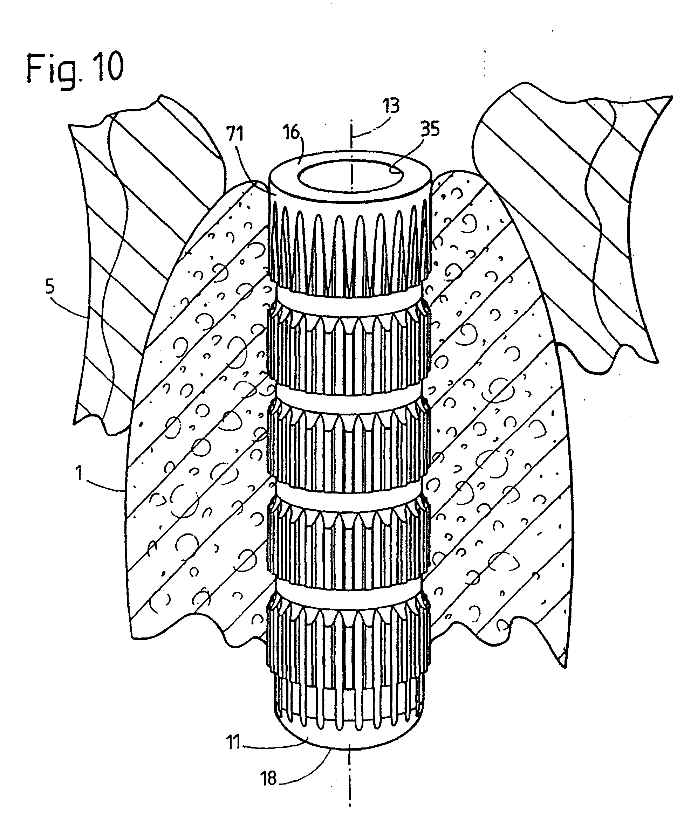

Das in Fig. 10 ersichtliche Implantat 1 ist weitgehend gleich ausgebildet wie das in den Figuren 8 und 9 dargestellte Implantat und unterscheidet sich von diesem nur dadurch, dass der eine glatte, zylindrische Umfangsfläche aufweisende Endabschnitt 71 nur sehr kurz ist. Das in Fig. 10 dargestellte Implantat 11 ist subgingival in den Knochen 1 im Mund eines Patienten eingesetzt. Das erste Implantatende 18 befindet sich wiederum am tiefsten im Knochen 1, während sich das zweite Implantatende 19 ungefähr in der Höhe des Knochenkamms und/oder nur wenig oberhalb des Knochens befindet. Das Implantat wird dann während der Einheilphase vollständig vom Weichgewebe bedeckt.

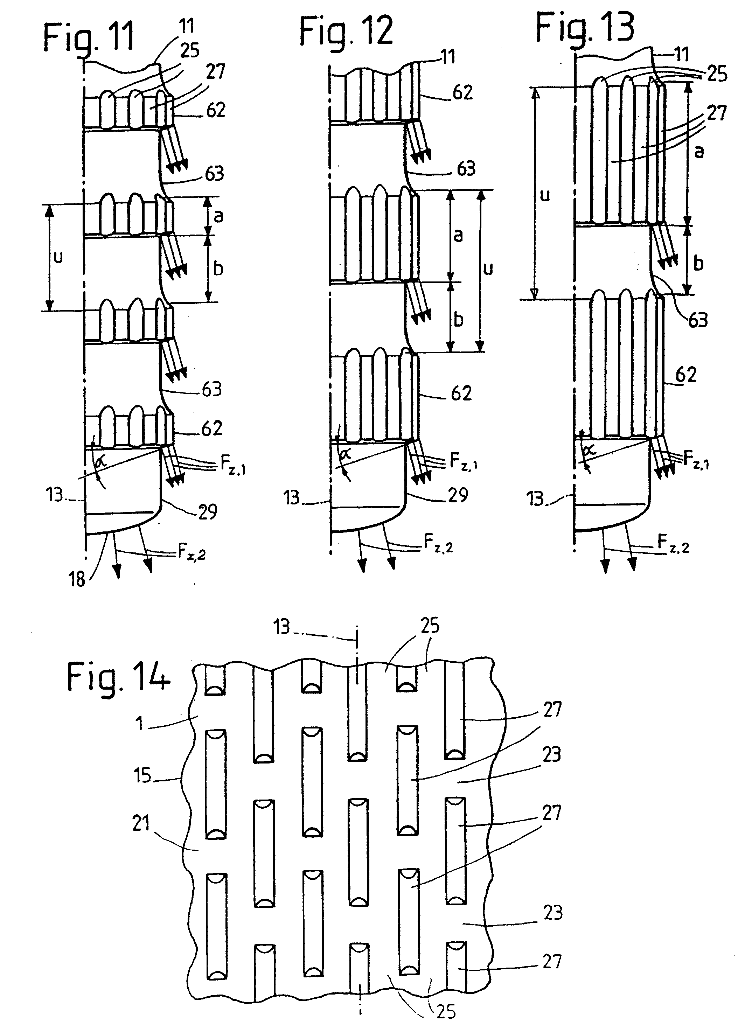

Die Figuren 11 bis 13 zeigen Teile der Verankerungspartien 15 verschiedener Implantate 11. Diese Verankerungspartien 15 sind ähnlich ausgebildet wie diejenigen der in den Figuren 8 bis 10 dargestellten Implantate und besitzen insbesondere ringförmige Bereiche 62 sowie zwischen diesen angeordnete Ringnuten 63. Die Bereiche 62 besitzen wiederum axiale Rillen 25 sowie Rippen 27. Die in den Figuren 11 bis 13 dargestellten Implantate 11 unterscheiden sich von den Implantaten gemäss den Figuren 8 bis 10 dadurch, dass sie zwischen dem ersten Implantatende 18 und dem sich am nächsten bei diesem befindenden, Rillen und Rippen aufweisenden Bereich 62 analog wie das in Fig. 1 gezeichnete Implantat einen Endabschnitt 29 mit einer vollständig glatten Zylinderfläche besitzen. Die unteren Endflächen der Rippen 27 der in den Figuren 11 bis 13 dargestellten Implantate bilden in Axialschnitten mit der Achse 13 einen Winkel α, der - wie auch bei den Implantaten gemäss den Figuren 8 bis 10 - etwa im gleichen Bereich liegt wie bei dem anhand der Figuren 1 bis 5 beschriebenen Implantat. Die oberen Endflächen der Rippen haben bei den in den Figuren 11 bis 13 dargestellten Implantaten und beispielsweise auch bei den Implantaten gemäss den Figuren 8 bis 10 einen konischen und also in Axialschnitten geraden Abschnitt, der durch einen in Axialschnitten konkav gebogenen Übergangsabschnitt stetig mit der zylindrischen Grundfläche der anschliessenden Ringnuten 63 verbunden ist.

In den Figuren 11 bis 13 sind noch die Entfernungen u von einander entsprechenden Stellen von zwei entlang der Achse aufeinander folgenden, ringförmigen Bereichen 62 eingetragen. Die Entfernung u entspricht mehr oder weniger der Steigung s des schraubenlinienförmigen Bereichs 22 des zuerst beschriebenen Implantats. Ferner sind in den Figuren 11 bis 13 die axialen Abmessungen a bzw. Längen der ringförmigen Bereiche 62 sowie Rippen 27 und die axialen Abmessungen b der Ringnuten 63 eingezeichnet. Die Abmessungen a, b werden dabei analog wie beim zuerst beschriebenen Implantat in der halben Höhe der Rippen gemessen. Die der Steigung eines Gewindes entsprechende Entfernung u beträgt vorzugsweise mindestens 1 mm, vorzugsweise höchstens 5 mm und zum Beispiel 1,5 mm bis 2,5 mm oder bis 3 mm. Die axiale Abmessung a bzw. Länge der Rippen kann etwa 20% bis 80% der Entfernung u betragen und kleiner oder grösser als die Abmessung b oder ungefähr gleich dieser sein. Die Figuren 11 bis 13 zeigen einige verschiedene Möglichkeiten für die Bemessung der Entfernung u sowie der Abstände a, b für einen gegebenen Durchmesser der Verankerungspartie.

In Fig. 14 ist ein Teil eines Implantats 11 in Abwicklung dargestellt. Das Implantat besitzt eine Verankerungspartie 15 mit einer im allgemeinen zylindrischen Umfangsfläche 21. Diese besitzt längliche, rippenartige Vorsprünge 27 mit einer zur Achse 13 parallelen Längsrichtung. Die Vorsprünge 27 oder Rippen sind in geraden, zur Achse parallelen Reihen angeordnet. Zwischen den zur gleichen Reihe gehörenden Vorsprüngen 27 sind Ausnehmungen 23 vorhanden. Die zu einander benachbarten Reihen gehörenden Vorsprünge sind parallel zur Achse 13 gegeneinander versetzt. Das obere Ende eines Vorsprungs 27 ragt zwischen zwei Vorsprünge der beiden benachbarten Vorsprungsreihen hinein. Das untere Ende des Vorsprungs ragt ebenfalls zwischen zwei Vorsprünge der beiden benachbarten Reihen hinein. Zwischen den Vorsprüngen von zwei einander benachbarten Reihen sind Ausnehmungen oder Zwischenräume 25 vorhanden, die zusammen eine zur Achse 13 parallele Rille bilden. An den beiden Enden eines Vorsprungs 27 sind vom Grund der Ausnehmungen 23 weg zur Mitte des Vorsprungs hin geneigte Endflächen vorhanden.

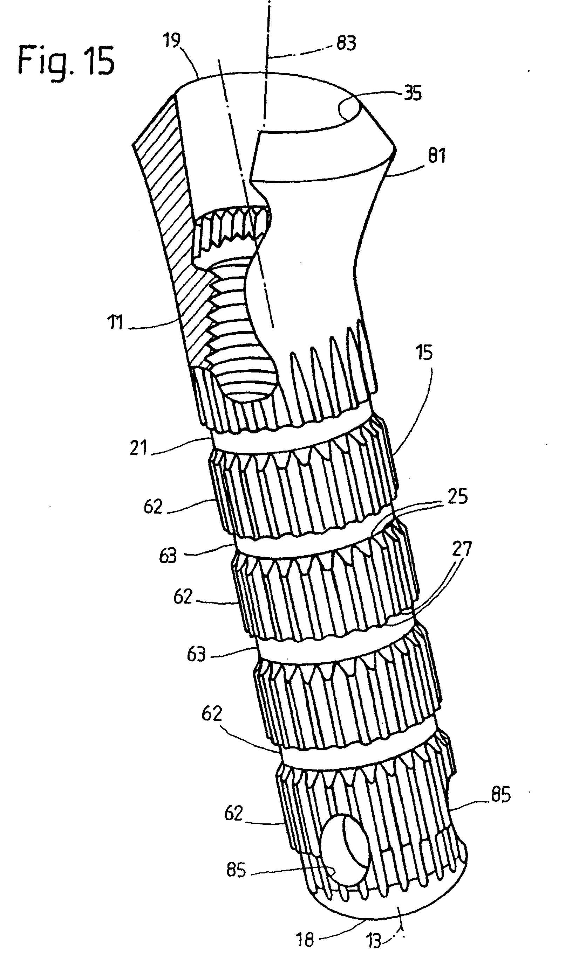

Das in Fig. 15 ersichtliche Dentalimplantat ist abgewinkelt und hat eine im allgemeinen zylindrische und zur Achse 13 koaxiale Verankerungspartie 15. Diese bildet das erste Implantatende 18 und hat eine Umfangsfläche 21. Das Implantat hat einen an das obere Ende der Verankerungspartie 15 anschliessenden Endabschnitt 81, der das zweite Implantatende 19 bildet und im wesentlichen koaxial zu einer Achse 83 ist, die mit der Achse 13 einen Winkel bildet. Das in das zweite Implantatende 19 mündende Sackloch 35 ist koaxial zur Achse 83 und besitzt unter anderem einen Abschnitt mit einem Innengewinde.

Die Verankerungspartie 15 hat ähnlich wie bei den Implantaten gemäss den Figuren 8 bis 13 ringförmige Bereiche 62, die durch Ringnuten 63 voneinander getrennt sind und entlang dem Umfang abwechselnd aufeinander folgende, zur Achse 13 parallele Rillen 25 und Rippen 27 haben. Das Implantat hat im unteren Teil der Verankerungspartie 15 ein zur Achse 13 koaxiales Sackloch, das in das erste Implantatende 18 mündet. Ferner hat das Implantat in der Nähe des ersten Implantatendes mindestens ein quer und radial zur Achse 13 verlaufendes Loch 85, das in das letztgenannte Sackloch mündet, wobei beispielsweise mindestens zwei um die Achse 13 herum verteilte Löcher 85 vorhanden sind. Die Löcher 85 können beispielsweise den untersten ringförmigen, Rillen und Rippen aufweisenden Bereich 62 durchdringen.

Die Verankerungspartie 15 des abgewinkelten Implantats kann analog wie bei den vorher beschriebenen, geraden Implantaten parallel zur Achse 13 in ein Loch eines Knochens hineingedrückt werden. In der Einheilphase kann der Knochen dann auch noch durch die Löcher 85 hindurchwachsen und in das in das erste Implantatende 18 mündende Sackloch hineinwachsen. Dadurch wird die Verankerung noch verbessert.

Damit das Implantat nicht zu stark durch quer zur Achse 13 verlaufende Löcher geschwächt wird, sollen jedoch nur wenige solche Löcher vorhanden sein. Ferner soll vorzugsweise mindestens etwa der am weitesten vom ersten Implantatende entfernte Drittel der Verankerungspartie frei von quer zur Achse 13 verlaufenden Löchern sein. Zudem soll die Verankerungspartie vorzugsweise mindestens einen ringförmigen Bereich 62 und besser mindestens zwei ringförmige Bereiche 62 aufweisen, bei denen die Umfangsfläche 21 kompakt und lochfrei ist.

Man könnte auch bei einem im wesentlichen gemäss der Fig. 1 ausgebildeten Implantat mit einem schraubenlinienförmigen Bereich 22 quer zur Achse 13 verlaufende Löcher vorsehen. Dann sollten jedoch ebenfalls nur im unteren Teil des Implantats solche Löcher vorhanden sein, so dass der schraubenlinienförmige Bereich 22 mindestens eine volle Schraubenwindung und besser mindestens zwei volle Schraubenwindungen aufweist, bei denen die Umfangsfläche 21 kompakt und lochfrei ist. Ähnliches gilt für ein Implantat mit gemäss der Fig. 14 angeordneten Vorsprüngen.

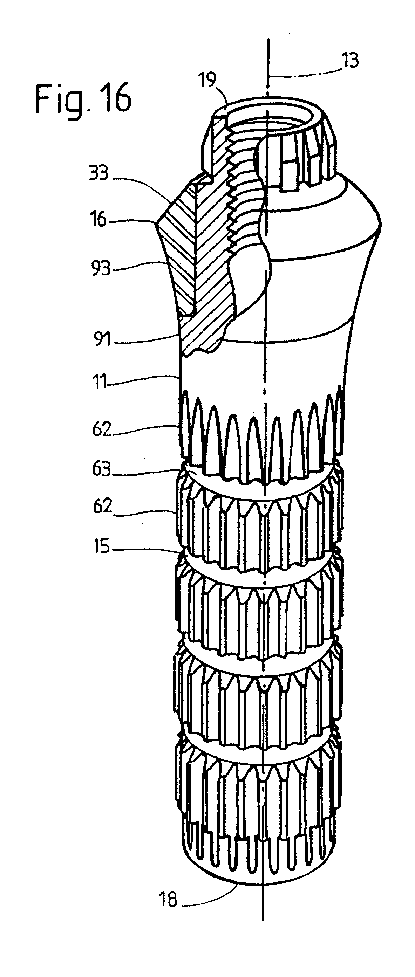

Das in der Fig. 16 ersichtliche Implantat 11 hat eine ähnliche äussere Form wie das Implantat gemäss der Fig. 1. Die Verankerungspartie 15 hat jedoch anstelle eines schraubenlinienförmigen Bereichs, mehrere ringförmige Bereiche 62, die durch Ringnuten 63 voneinander getrennt sind und axiale Rillen und Rippen aufweisen. Das in Fig. 16 ersichtliche Implantat besitzt einen metallischen, nämlich aus Titan bestehenden, einstückigen Hauptkörper 91, der sich vom ersten Implantatende 18 bis zum zweiten Implantatende 19 erstreckt. Das Implantat besitzt ferner einen ringförmigen Keramikkörper 93, der die Schulter 16 mit der ringförmigen Schulterfläche 33 bildet. Der Keramikkörper 93 sitzt in einer ringförmigen Kehle des metallischen Hauptkörpers und ist beispielsweise durch eine Kleb- oder Lötverbindung starr und im wesentlichen unlösbar mit dem Hauptkörper verbunden.

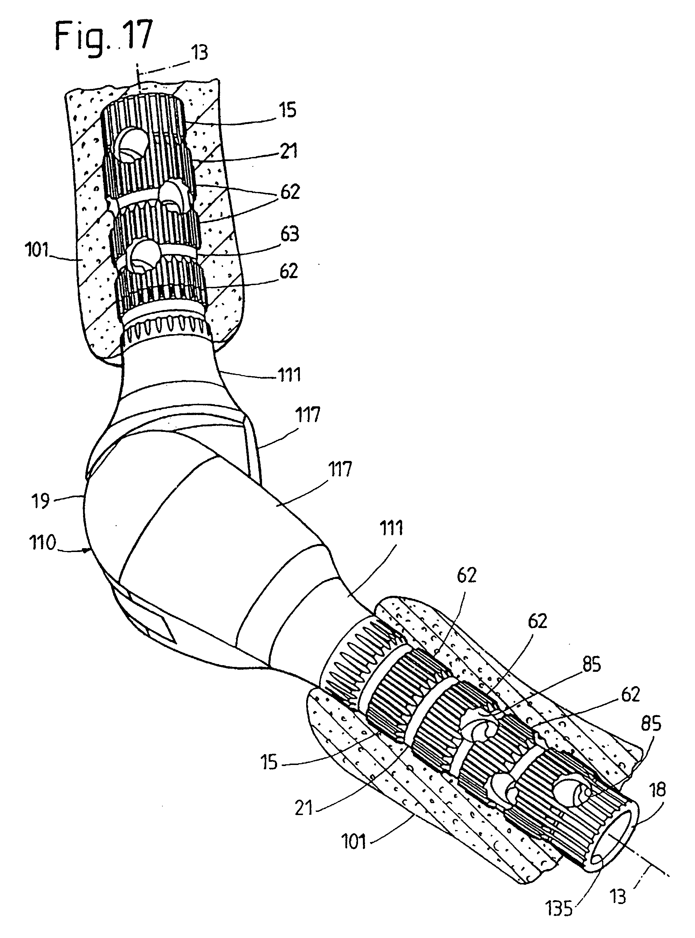

In der Fig. 17 sind zwei Knochen 101 eines Fingers eines Patienten und ein künstliches Fingergelenk 110 ersichtlich. Das Fingergelenk 110 besitzt zwei Implantate 111. Jedes Implantat 111 besitzt eine Verankerungspartie 15 und einen Gelenkteil oder Gelenkkopf 117. Jede Verankerungspartie besitzt eine im allgemeinen zylindrische Umfangsfläche 21. Diese besitzt beispielsweise einige ringförmige Bereiche 62, die durch Ringnuten 63 voneinander getrennt sowie mit axialen Rillen 25 und Rippen 27 versehen sind. Jede Verankerungspartie 15 hat ein axiales Loch 135, das in das von der Verankerungspartie gebildete, erste Implantatende 18 mündet. Das zweite Implantatende 19 wird durch den Gelenkteil 117 gebildet. Jede Verankerungspatie ist durch eine metallische, zum Beispiel aus Titan bestehende Hülse mit einem durchgehenden axialen Loch gebildet. Die beiden Gelenkteile bestehen aus einem etwas elastisch deformierbaren Kunststoff, haben ineinander einklipsbare Schwenklagermittel und sind starr mit der zum betreffenden Implantat gehörenden Verankerungspartie verbunden. Jede Verankerungspartie hat noch einige quer zur Achse 13 verlaufende, in das axiale Loch 135 mündende Löcher 85.

Soweit in den Beschreibungen der Figuren 8 bis 16 nichts anderes geschrieben wurde, können die in diesen Figuren dargestellten Implantate ähnliche Eigenschaften haben, wie das bzw ein vorher beschriebenes Implantat.

Des weiteren können Merkmale veschiedener Implantate miteinander kombiniert werden. Beispielsweise könnten die ringförmigen Bereiche 62 der in den Figuren 8, 10, 15 bis 17 dargestellten Implantate durch einen schraubenlinienförmigen Bereich 22 ersetzt werden. Des weiteren kann die obere Endfläche des in Fig. 1 ersichtlichen, schraubenlinienförmigen Bereichs 22 analog wie die oberen Endflächen der ringförmigen Bereiche 62 der Figuren 11 bis 13 im Axialschnitt teils gerade und teils konkav gebogen sein. Ferner könnte man eine Verankerungspartie vorsehen, die mehr als einen schraubenlinienförmigen Bereich 22 und mehr als eine schraubenlinienförmige Nut, beispielsweise zwei oder drei solche Bereiche und Nuten aufweist und analog zu einem mehrgängigen Gewinde ausgebildet ist.

## Patentansprüche

1. Implantat zum Halten und/oder Bilden eines Zahnersatzes oder eines künstlichen Fingergelenks (110), mit einer zum Verankern in einen Knochen (1, 101) bestimmten Verankerungspartie (15), die ein zum Einsetzen in den Knochen (1, 101) bestimmtes Implantatende (18) bildet sowie eine Achse (13) und eine diese umschliessende Umfangsfläche (21) mit um die Achse (13) herum verteilten, von dieser wegragenden Vorsprüngen (27) aufweist, wobei mindestens der grösste Teil der Vorsprünge (27) mindestens ein dem genannten Implantatende (18) zugewandtes und/oder ein dem genannten Implantatende (18) abgewandte Ende hat, das an eine Ausnehmung (23, 63) angrenzt, **dadurch gekennzeichnet,**
**dass** mindestens der grösste Teil der Vorsprünge (27) zur Achse (13) parallele Scheitel aufweist und/oder dass Scheitel von entlang der Achse (13) gegeneinander versetzten Vorsprüngen (27) gleiche Abstände von der Achse (13) haben.

2. Implantat nach Anspruch 1, **dadurch gekennzeichnet, dass** mindestens der grösste Teil der Vorsprünge (27) länglich ist und eine zur Achse (13) parallele Längsrichtung sowie mindestens über den grössten Teil der Vorsprungs-Länge eine konstante Querschnittsform hat und eine konstante Querschnittsabmessung hat.

3. Implantat nach Anspruch 1 oder 2, **dadurch gekennzeichnet, dass** die Vorsprünge (27) um die Achse (13) herum verteilte, zu dieser parallele Reihen bilden und dass mindestens alle zur gleichen Reihe gehörenden und sich zwischen anderen Vorsprüngen (27) dieser Reihe befindenden Vorsprünge (27) gleiche Querschnittsabmessungen und gleiche Querschnittsformen haben.

4. Implantat nach einem der Ansprüche 1 bis 3, **dadurch gekennzeichnet, dass** die Scheitel der Vorsprünge (27) zusammen eine Zylinderfläche definieren und dass die bzw. jede Ausnehmung (23) einen von der Umfangsfläche (21) gebildeten Grund hat, der beispielsweise mindestens zum Teil zylindrisch ist.

5. Implantat nach Anspruch 4, **dadurch gekennzeichnet, dass** mindestens der grösste Teil der Vorsprünge (27) an dem sich näher beim genannten Implantatende (18) befindenden Ende eine Endfläche hat, die mit der Achse (13) einen Winkel von mindestens 60° bildet und beispielsweise von der Achse (13) und vom genannten Implantatende (18) weggeneigt ist und beispielsweise durch mindestens eine Kante von mindestens einer zur Achse (13) parallelen Fläche des Vorsprungs (27) abgegrenzt ist.

6. Implantat nach einem der Ansprüche 1 bis 5, **dadurch gekennzeichnet, dass** die Vorsprünge (27) durch mindestens eine schraubenlinienförmige, um die Achse (13) herum verlaufende Ausnehmung (23, 63) voneinander getrennt sind, wobei die schraubenlinienförmige Ausnehmung (23) vorzugsweise mindestens zwei ganze Windungen und zum Beispiel mindestens drei ganze Windungen bildet.

7. Implantat nach einem der Ansprüche 1 bis 5, **dadurch gekennzeichnet, dass** die Vorsprünge (27) durch mindestens eine ringförmige, um die Achse (13) herum verlaufende Ausnehmung (23, 63) voneinander getrennt sind, wobei vorzugsweise mindestens zwei und beispielsweise mindestens drei zwischen Vorsprüngen (27) angeordneten, voneinander entlang der Achse (13) in Abstand stehende Ausnehmungen (23, 63) vorhanden sind.

8. Implantat nach einem der Ansprüche 1 bis 5, **dadurch gekennzeichnet, dass** in axialer Richtung gegeneinander versetzte Vorsprünge (27) zwischen einander hineinragen.

9. Implantat nach einem der Ansprüche 1 bis 8, **dadurch gekennzeichnet, dass** jeder Vorsprung (27) in einem zur Achse (13) rechtwinkligen Querschnitt zwei einander abgewandte Flanken und einen diese stetig miteinander verbindenden, im Querschnitt gebogenen Scheitel hat.

10. Implantat nach einem der Ansprüche 1 bis 9, **dadurch gekennzeichnet, dass** entlang einer die Achse (13) umschliessenden Schraubenwindung und/oder entlang einem die Achse (13) umschliessenden Kreis mindestens 10 und vorzugsweise mindestens 15 Vorsprünge (27) vorhanden sind, wobei ein die Vorsprünge (27) enthaltender Abschnitt der Verankerungspartie (15) eine Oberfläche hat, die vorzugsweise mindestens 30% grösser ist als eine zylindrische Hüllfläche, die sich über die axiale Ausdehnung des genannten Abschnitts der Verankerungspartie (15) erstreckt und sich an die Scheitel der Vorsprünge (27) anschmiegt, und wobei die Verankerungspartie (15) vorzugsweise mindestens einen die Achse (13) umschliessenden Bereich (62) von Vorsprüngen (27) oder mindestens eine sich über eine volle Schraubenlinienwindung erstreckenden Gruppe von Vorsprüngen (27) aufweist, bei denen die Umfangsfläche (21) kompakt und lochfrei ist.

11. Implantat nach einem der Ansprüche 1 bis 10, **dadurch gekennzeichnet, dass** die Verankerungspartie (15) einen im Allgemeinen zylindrischen, mit den Vorsprüngen (27) versehenen Abschnitt aufweist, der zusammen mit dem genannten Implantatende (18) und den Vorsprüngen (27) aus einem einstückigen Körper gebildet ist, der vorzugsweise aus einem metallischen Material besteht, wobei die Verankerungspartie (15) zum Beispiel vollständig durch den genannten, einstückigen Körper gebildet ist oder zum Beispiel einen den genannten, einstückigen Körper bildenden, metallischen Hauptkörper (91) und einen ringförmigen, eine Schulter (16) bildenden Keramikkörper (93) aufweist.

12. Implantat nach einem der Ansprüche 1 bis 11, **dadurch gekennzeichnet, dass** die Verankerungspartie (15) einen die Vorsprünge (27) aufweisenden, im Allgemeinen zylindrischen Abschnitt hat, dass die Vorsprünge (27) mindestens 10 und beispielsweise mindestens 15 zur Achse (13) parallele, um diese herum verteilte Rippen bilden, von denen jede mindestens zwei Vorsprünge (27) hat, die durch eine Ausnehmung (23, 63) voneinander getrennt und durch zur Achse (13) parallele Rippen gebildet sind.

## Claims

1. Implant for holding and/or forming a dental prosthesis or artificial finger joint (110), with an anchoring part (15) intended to be anchored in a bone (1, 101) and forming an implant end (18) intended for insertion into the bone (1, 101), and an axis (13), and a peripheral surface (21) surrounding this axis, with protuberances (27) which are distributed around the axis (13) and project away from it, at least the majority of the protuberances (27) having at least an end directed toward said implant end (18) and/or an end directed away from said implant end (18) and contiguous with a recess (23, 63), **characterized in that** at least the majority of the protuberances (27) have apices parallel with the axis (13) and/or **in that** apices of protuberances (27) mutually staggered along the axis (13) are at identical distances from the axis (13).

2. Implant according to Claim 1, **characterized in that** at least the majority of the protuberances (27) are elongate and have a longitudinal direction parallel with the axis (13), and they have a constant cross-sectional shape and a constant cross-sectional size at least over the greatest part of the length of protuberances.

3. Implant according to Claim 1 or 2, **characterized in that** the protuberances (27) form rows distributed around the axis (13) and parallel with it, and **in that** at least all the protuberances (27) belonging to the same row and situated between other protuberances (27) of this row have identical cross-sectional dimensions and identical cross-sectional shapes.

4. Implant according to one of Claims 1 to 3, **characterized in that** the apices of the protuberances (27) together define a cylinder surface, and **in that** the or each recess (23) has a base which is formed by the peripheral surface (21) and which is for example at least partially cylindrical.

5. Implant according to Claim 4, **characterized in that** at least the majority of the protuberances (27), at the end situated nearer to said implant end (18), have a terminal surface which forms with the axis (13) an angle of at least 60° and is for example inclined away from the axis (13) and from said implant end (18) and is for example delimited by at least one edge of at least one surface of the protuberance (27) parallel with the axis (13).

6. Implant according to one of Claims 1 to 5, **characterized in that** the protuberances (27) are separated from each other by at least one helical recess (23, 63) extending around the axis (13), the helical recess (23) preferably forming at least two complete windings and for example at least three complete windings.

7. Implant according to one of Claims 1 to 5, **characterized in that** the protuberances (27) are separated from each other by at least one annular recess (23, 63) extending around the axis (13), preferably at least two and for example at least three recesses (23, 63) being arranged between protuberances (27) and spaced apart from each other along the axis (13).

8. Implant according to one of Claims 1 to 5, **characterized in that** protuberances (27) mutually staggered in the axial direction project in between each other.

9. Implant according to one of Claims 1 to 8, **characterized in that** each protuberance (27) has, in a cross section at right angles to the axis (13), two flanks directed away from each other and an apex of curved cross section continuously connecting these to each other.

10. Implant according to one of Claims 1 to 9, **characterized in that** at least 10 and preferably at least 15 protuberances (27) are present along a helical winding enclosing the axis (13) and/or along a circle enclosing the axis (13), where a section of the anchoring part (15) containing the protuberances (27) has a surface which is preferably at least 30% greater than a cylindrical enveloping surface, which extends along the axial extent of said section of the anchoring part (15) and hugs the apices of the protuberances (27), and where the anchoring part (15) has preferably at least one area (62) of protuberances (27) enclosing the axis (13) or at least one group of protuberances (27) extending through a complete helical winding, in which the peripheral surface (21) is compact and free from holes.

11. Implant according to one of Claims 1 to 10, **characterized in that** the anchoring part (15) has a generally cylindrical portion which is provided with the protuberances (27) and which together with said implant end (18) and the protuberances (27) is formed from a one-piece body which is preferably made of a metal, the anchoring part (15) being formed, for example, completely by said one-piece body or having, for example, a metal main body (91), forming said one-piece body, and an annular ceramic body (93) forming a shoulder (16).

12. Implant according to one of Claims 1 to 11, **characterized in that** the anchoring part (15) has a generally cylindrical portion having the protuberances (27), and **in that** the protuberances (27) form at least 10 and for example at least 15 ribs which are parallel to the axis (13) and are distributed about the latter, each of which ribs has at least two protuberances (27) which are separated from one another by a recess (23, 63) and are formed by ribs parallel to the axis (13).

## Revendications

1. Implant pour maintenir et/ou former une prothèse dentaire et/ou une articulation artificielle du doigt (110), avec une partie d'ancrage (15) destinée à être ancrée dans un os (1, 101) formant une extrémité d'implant (18) destinée à être implantée dans l'os (1, 101) et présentant un axe (13) et une surface périphérique (21) entourant celui-ci, avec des parties saillantes (27) réparties autour de l'axe (13) et s'éloignant de celui-ci vers l'extérieur, au moins la majeure partie des parties saillantes (27) ayant au moins une extrémité orientée vers ladite extrémité d'implant (18) et/ou une extrémité orientée à l'opposé de ladite extrémité d'implant (18) adjacente à une gorge (23, 63), **caractérisé en ce qu'**au moins la majeure partie des parties saillantes (27) présente des sommets parallèles à l'axe (13) et/ou **en ce que** des sommets de parties saillantes (27) décalées les unes par rapport aux autres le long de l'axe (13) ont la même distance par rapport l'axe (13).

2. Implant selon la revendication 1, **caractérisé en ce qu'**au moins la majeure partie des parties saillantes (27) est de forme allongée et a une orientation longitudinale parallèle à l'axe (13) ainsi qu'une forme de section constante sur au moins la majeure partie de la longueur de la partie saillante, et présente une dimension de section constante.

3. Implant selon la revendication 1 ou 2, **caractérisé en ce que** les parties saillantes (27) forment des rangées réparties autour de l'axe (13), parallèles à celui-ci, et **en ce qu'**au moins toutes les parties saillantes (27) appartenant à la même rangée et se trouvant entre d'autres parties saillantes (27) de cette rangée ont des sections de dimension et de forme identiques.

4. Implant selon l'une des revendications 1 à 3, **caractérisé en ce que** les sommets des parties saillantes (27) définissent ensemble une surface cylindrique et **en ce que** la ou chaque gorge (23) possède un fond formé par la surface périphérique (21), qui est par exemple au moins en partie cylindrique.

5. Implant selon la revendication 4, **caractérisé en ce qu'**au moins la majeure partie des parties saillantes (27) possède une surface d'extrémité à l'extrémité située le plus près de ladite extrémité d'implant (18) formant un angle d'au moins 60° avec l'axe (13) et étant par exemple inclinée en s'éloignant de l'axe (13) et de ladite extrémité d'implant (18) et étant par exemple délimitée par au moins une arête d'au moins une surface de la partie saillante (27) parallèle à l'axe (13).

6. Implant selon l'une des revendications 1 à 5, **caractérisé en ce que** les parties saillantes (27) sont séparées les unes des autres par au moins une gorge (23, 63) en forme de pas de vis s'étendant autour de l'axe (13), la gorge (23) en forme de pas de vis formant de préférence au moins deux tours complets et, par exemple, au moins trois tours complets.

7. Implant selon l'une des revendications 1 à 5, **caractérisé en ce que** les parties saillantes (27) sont séparées les unes des autres par au moins une gorge (23, 63) annulaire s'étendant autour de l'axe (13), de préférence au moins deux et par exemple au moins trois gorges (23, 63) étant' disposées de manière espacée les unes des autres le long de l'axe (13) entre les parties saillantes (27).

8. Implant selon l'une des revendications 1 à 5, **caractérisé en ce que** des parties saillantes (27) décalées entre elles dans le sens axial pénètrent les unes entre les autres.

9. Implant selon l'une des revendications 1 à 8, **caractérisé en ce que** chaque partie saillante (27) présente, dans une section perpendiculaire à l'axe (13), deux flancs s'éloignant l'un de l'autre et un sommet de section courbe reliant ceux-ci de manière continue.

10. Implant selon l'une des revendications 1 à 9, **caractérisé en ce qu'**il y a au moins 10 et de préférence au moins 15 parties saillantes (27) le long d'un pas de vis entourant l'axe (13) et/ou le long d'un cercle entourant l'axe (13), une section de la partie d'ancrage (15) qui contient les parties saillantes (27) présentant une surface qui est de préférence plus grande d'au moins 30% qu'une surface enveloppante cylindrique s'étendant sur l'extension axiale de ladite section de la partie d'ancrage (15) et épousant les sommets des parties saillantes (27), la partie d'ancrage (15) présentant de préférence au moins une zone (62) de parties saillantes (27) entourant l'axe (13) ou au moins un groupe de parties saillantes (27) s'étendant sur un tour complet de pas de vis, dans laquelle la surface périphérique (21) est compacte et ne présente pas de trou.

11. Implant selon l'une des revendications 1 à 10, **caractérisé en ce que** la partie d'ancrage (15) présente une section sensiblement cylindrique munie des parties saillantes (27) formée, avec ladite extrémité d'implant (18) et les parties saillantes (27), d'un corps monobloc réalisé de préférence dans un matériau métallique, la partie d'ancrage (15) étant par exemple entièrement formée par ledit corps monobloc ou présentant par exemple un corps principal (91) métallique formant ledit corps monobloc et un corps annulaire (93) céramique formant un épaulement (16).

12. Implant selon l'une des revendications 1 à 11, **caractérisé en ce que** la partie d'ancrage (15) présente une section sensiblement cylindrique munie des parties saillantes (27), **en ce que** les parties saillantes (27) forment au moins 10 et par exemple au moins 15 nervures parallèles à l'axe (13) et réparties autour de celui-ci, dont chacune présente au moins deux parties saillantes (27) séparées les unes des autres par une gorge (23, 63) et constituées par des nervures parallèles à l'axe (13).
